(19) Europäisches Patentamt
European Patent Office
Office européen des brevets

(11) **EP 3 683 800 A1**

(12) **EUROPEAN PATENT APPLICATION**

(43) Date of publication:
**22.07.2020 Bulletin 2020/30**

(51) Int Cl.:
***G16H 50/50*** *(2018.01)*

(21) Application number: **20152376.8**

(22) Date of filing: **17.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2019 IN 201921002217**

(71) Applicant: **Tata Consultancy Services Limited Maharashtra (IN)**

(72) Inventors:
- **Jayabal, Hemalatha**
  **411013 Pune - Maharashtra (IN)**
- **Verma, Nitu**
  **411013 Pune - Maharashtra (IN)**

- **Sharma, Paramveer**
  **411013 Pune - Maharashtra (IN)**
- **Gajula, Kishore**
  **411013 Pune - Maharashtra (IN)**
- **Dingari, Naga Neehar**
  **411013 Pune - Maharashtra (IN)**
- **Badhe, Yogesh Kailas**
  **411013 Pune - Maharashtra, (IN)**
- **Gupta, Rakesh**
  **411013 Pune - Maharashtra (IN)**
- **Rai, Beena**
  **411013 Pune - Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR INSILICO DESIGN AND TESTING OF FORMULATIONS USING PHYSIOCHEMICAL SKIN MODEL**

(57)    There is a lack of knowledge about bridging the gap between the length and time scales of multiple phenomena related to transport across skin layers and structural changes in skin proteins (collagen, elastin, etc.). The embodiments herein provide a method and system for insilico design and testing of formulations using a physiochemical skin model, which provides a technical solution of integrating expertise in molecular modelling and macroscopic modelling techniques to provide a complete solution for formulation design. The present disclosure integrates phenomena's happening at different length and time scales using molecular, microscopic and macroscopic modelling techniques. The present subject matter incorporates expertise in diverse modelling techniques such as molecular modelling and macroscopic modelling and knowledge about bio-molecular systems and mechanical characterization of materials.

FIG. 2

EP 3 683 800 A1

## Description

### CROSS-REFERENCE TO RELATED APPLICATIONS AND PRIORITY

The present application claims priority from Indian provisional patent application no. IN-201921002217, filed on January 18, 2019.

### TECHNICAL FIELD

The disclosure herein generally relates to insilico design and testing using skin modelling, and, more particularly to method and system for insilico design and testing of formulations using physiochemical skin model.

### BACKGROUND

In general, chemical sensing refers to chemical alteration of the skin microscopic components (like skin proteins and lipids), whereas mechanical sensing refers to altering the overall mechanical properties of the skin (like viscoelasticity, etc.). Also, in each sensing methodology, most of the existing technologies do not combine the phenomena that occurs at different length and time scales. For instance, skin's mechanical behavior has been modelled separately at different scales but a comprehensive multi-scale model that explains the viscoelastic nature of skin and bridging the different scales is not available. Further, it is not fully understood how microscopic changes in skin (chemical structural changes of underlying molecule/s due to age, location and uses of cosmetics/formulations etc.) effect its mechanical properties. These understandings would enable design of effective formulations to alter microscopic skin properties and to obtain anti-ageing effects. Also, current methods/technologies lack at combining the chemical and mechanical sensing of cosmetics or formulations.

### SUMMARY

Embodiments of the present disclosure present technological improvements as solutions to one or more of the above-mentioned technical problems recognized by the inventors in conventional systems. For example, in one embodiment, there is provided a method for insilico design and testing of formulations. The method comprises iteratively performing steps until a formulation provides predefined effects on a physiochemical skin model in accordance to a set of skin conditions, wherein the formulation is a combination among a plurality of combinations generated from a plurality of active ingredients, a plurality of excipients, a plurality of solvents. The steps comprising: designing the formulation to be tested using the physiochemical model of one or more skin layers for a skin application of interest, wherein designing comprises generating the combination for the formulation comprising a solvent selected among the plurality of solvents, one or more active ingredients selected among the plurality of active ingredients, and one or more excipients selected among the plurality of excipients. The physiochemical model comprises: a plurality of chemical models of the one or more skin layer comprising multiscale chemical models of the one or more skin layer ranging from a molecular scale to a macro scale; and a plurality of mechanical models of the one or more skin layers comprising multiscale mechanical models of the one or more skin layers ranging from the molecular scale to the macro scale. The steps further comprise determining whether the combination of the selected one or more active ingredients, the selected one or more excipients and the selected one or more solvents satisfies a stability criteria, wherein the combination is modified if the stability criteria is not satisfied. Thereafter, selecting at least one of a chemical model among the plurality of chemical models and a mechanical model among the plurality of mechanical models in accordance with the skin application of interest, if the formulation satisfies the stability criteria. Further, performing an insilico testing of the to generate test results using at least one of: the chemical model in at least one of the molecular scale and the macro scale to determine whether the formulation satisfies a skin transport property criteria; and the mechanical model in at least one of the molecular and the macro scale to determine whether the formulation satisfies a skin mechanical property criteria. Furthermore the steps comprise analyzing the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

In yet another aspect, there is provided a system for insilico design and testing of formulations. The system comprising a memory storing instructions; one or more Input/output (I/O) interfaces; and processor(s) coupled to the memory via the one or more I/O interfaces, wherein the processor(s) is configured by the instructions to iteratively perform steps until a formulation provides predefined effects on a physiochemical skin model in accordance to a set of skin conditions, wherein the formulation is a combination among a plurality of combinations generated from a plurality of active ingredients, a plurality of excipients, and a plurality of solvents. The steps comprising: designing the formulation to be tested using the physiochemical model of one or more skin layers for a skin application of interest, wherein designing comprises generating the combination for the formulation comprising a solvent selected among the plurality of solvents, one or more active ingredients selected among the plurality of active ingredients, and one or more excipients selected among the plurality of excipients. The physiochemical model comprises: a plurality of chemical models of the one or more skin layer comprising multiscale chemical models of the one or more skin layer ranging from a molecular scale to a macro scale; and a plurality of mechanical models of the one or more skin layers comprising multiscale mechanical models of the one or more skin layers ranging from the molecular scale to the macro scale. Further, the steps comprise determining whether the combination of the selected one or more active ingredients, the selected one or more excipients and the selected one or more solvents satisfies a stability criteria, wherein the combination is modified if the stability criteria is not satisfied. Further, the steps comprise selecting at least one of a chemical model among the plurality of chemical models and a mechanical model among the plurality of mechanical models in accordance with the skin application of interest, if the formulation satisfies the stability criteria and performing an insilico testing of the to generate test results using at least one of: the chemical model in at least one of the molecular scale and the macro scale to determine whether the formulation satisfies a skin transport property criteria; and the mechanical model in at least one of the molecular and the macro scale to determine whether the formulation satisfies a skin mechanical property criteria. Furthermore, the steps comprise analyzing the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

In yet another aspect, there are provided one or more non-transitory machine readable information storage mediums comprising one or more instructions, which when executed by one or more hardware processors causes a method for insilico design and testing of formulations. The method comprises iteratively performing steps until a formulation provides predefined effects on a physiochemical skin model in accordance to a set of skin conditions, wherein the formulation is a combination among a plurality of combinations generated from a plurality of active ingredients, a plurality of excipients, and a plurality of solvents. The steps comprising: designing the formulation to be tested using the physiochemical model of one or more skin layers for a skin application of interest, wherein designing comprises generating the combination for the formulation comprising a solvent selected among the plurality of solvents, one or more active ingredients selected among the plurality of active ingredients, and one or more excipients selected among the plurality of excipients. The physiochemical model comprises: a plurality of chemical models of the one or more skin layer comprising multiscale chemical models of the one or more skin layer ranging from a molecular scale to a macro scale; and a plurality of mechanical models of the one or more skin layers comprising multiscale mechanical models of the one or more skin layers ranging from the molecular scale to the macro scale. The steps further comprise determining whether the combination of the selected one or more active ingredients, the selected one or more excipients and the selected one or more solvents satisfies a stability criteria, wherein the combination is modified if the stability criteria is not satisfied. Thereafter, selecting at least one of a chemical model among the plurality of chemical models and a mechanical model among the plurality of mechanical models in accordance with the skin application of interest, if the formulation satisfies the stability criteria. Further, performing an insilico testing of the to generate test results using at least one of: the chemical model in at least one of the molecular scale and the macro scale to determine whether the formulation satisfies a skin transport property criteria; and the mechanical model in at least one of the molecular and the macro scale to determine whether the formulation satisfies a skin mechanical property criteria. Furthermore the steps comprise analyzing the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a block diagram of a system for insilico design and testing of formulations using a physiochemical model of skin layer/s, interchangeably referred as physiochemical skin model, in accordance with some embodiments of the present disclosure.
FIG. 2 is a functional block diagram of the system of FIG. 1 illustrating insilico design and testing of formulations using physiochemical model of skin layer/s, in accordance with some embodiments of the present disclosure.
FIG. 3 is a functional block diagram illustrating a formulations design module of the system of FIG. 1, in accordance with some embodiments of the present disclosure.
FIG. 4 is a functional block diagram illustrating a chemical model of the system of FIG. 1, in accordance with some embodiments of the present disclosure.
FIG. 5 is a functional block diagram illustrating a mechanical model of the system of FIG. 1, in accordance with some embodiments of the present disclosure.

FIGS. 6A and 6B depict a flow diagram illustrating a method for insilico design and testing of skin formulations using the physiochemical model generated by the system of FIG. 1, in accordance with some embodiments of the present disclosure.

FIG. 7 is a graphical representation of a Generalized Voigt model explaining creep behavior, in accordance with some embodiments of the present disclosure.

FIGS. 8A and 8B are a graphical representation of model parameters with respect to age and location respectively, in accordance with an example embodiment of the present disclosure.

FIG. 9 is a graphical representation of comparison of hysteretic behavior between model and experimental data, in accordance with some embodiments of the present disclosure.

FIG. 10 is a graphical representation of Generalized Maxwell model of experimental data for a single cycle, in accordance with some embodiments of the present disclosure.

FIG. 11 is a graphical representation of the Stress-Strain behavior of a representative collagen molecule (Type III) wherein the rupture behavior of collagen molecule was studied using molecular simulations, in accordance with some embodiments of the present disclosure.

FIGS. 12 through 14 depict building of chemical models and examples of chemical models, in accordance with some embodiments of the present disclosure.

FIGS. 15 and FIG. 16 illustrate experimental results when a specific selected chemical model, at molecular scale and macro scale, is used by the system of FIG. 1 for testing formulation comprising polyethylene glycol (PEG), in accordance with some embodiments of the present disclosure.

FIG. 17 illustrates hierarchal or multiscale arrangement of skin dermis component (collagen) which is responsible for skin's viscoelastic or mechanical properties.

FIG. 18 illustrates simulation results of system of FIG. 1 indicating structural changes to collagen molecules when stress is applied, in accordance to some embodiments of the present disclosure.

FIG. 19 illustrates a force-strain diagram of collagen molecule, with and without PEG, compared with molecular dynamics simulation (MDS) with reference to FIG. 18, in accordance to some embodiments of the present disclosure.

FIGS. 20, through 24 illustrate experimental results when a specific selected mechanical model of the system of FIG. 1 is used for testing formulation comprising the PEG, in accordance with some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

Existing techniques of skin modelling hardly attempt to provide a complete framework for design of formulations starting from cosmetic delivery to cosmetic efficacy. There is a lack of knowledge about bridging the gap between the length and time scales of multiple phenomena related to transport across skin layers and structural changes in skin proteins (e.g., collagen, elastin, etc.). There are no preliminary/extensive molecular understandings of the effect of cosmetics in the skin that bring about the change in mechanical properties of skin.

The present disclosure provides a technical solution of integrating expertise in molecular modelling and macroscopic modelling techniques to provide a complete solution for formulation design. The present subject matter also acts as a screening tool for various formulations thereby reducing the time and effort taken in studying efficacy of cosmetics using in vitro/in-vivo studies. The present disclosure integrates phenomena's happening at different length and time scales using molecular, microscopic and macroscopic modelling techniques. The present subject matter incorporates expertise in diverse modelling techniques such as molecular modelling and macroscopic modelling and knowledge about bio-molecular systems and mechanical characterization of materials. For example, the current technique is to conduct in vitro tests to study the efficacy of drug formulations. These techniques are expensive, and the results do not translate well to in vivo conditions in human body. The present subject matter over comes the aforementioned technical difficulties. The method of the present disclosure provides an in-silico framework to study the stability, permeability, release profiles, efficacy of the cosmetic and more particularly, to methods, systems and computer program products for simulating constituents of skin layers (for example, lipid membrane and corneocytes of stratum corneum, protein of dermis layer (collagen, elastin etc.) to name a few). The model and methodology are validated with experimental data and can be used in the design and virtual testing/screening of complex cosmetics/formulations. Herein, the skin referred is a multilayer and complex organ of a body (human). In general, skin layers are made up of various kind of biomolecules (lipids, proteins, receptors, etc. to name few). For example, the skin's top layer, stratum corneum, is made up of more than 300 different types of biomolecules. Modelling of all these molecules is not possible with current computing architecture. Hence, to overcome the problem, in the present disclosure a simple model of skin was first developed, having each class of biomolecules reduced and tested rigorously in various cases. Since the model is validated, more complexity can be added based on the application/s. The method of the present disclosure is alternatively referred as integrated in-silico chemical and mechanical model.

Often, skin models provided by the existing methods in art model the skin independently as a) a chemical model focused on transport properties such as permeation and the like or b) a physical model focused on mechanical properties of skin such as viscoelasticity and the like. However, to understand end to end effect of a formulation being tested for skin, integration of the two models is essential and the same is provided by the method and system disclosed herein, by creating a more realistic virtual or insilico model of skin. Such an integration enables more rapid and accurate result during insilico testing of skin formulations for healthcare and personal care applications. Integrating the independent chemical and mechanical models is challenging, since this demands expertise from the domain of mechanical engineering, chemical engineering, solid mechanics, materials engineering. Also, required are methods from various domains (molecular modelling, finite element methods, computational fluid dynamics, structural mechanics, RVE, and the like). Connecting physics at the molecular level to all the way up to macroscopic skin level is a challenging technical problem.

Further, a standard chemical model or a standard mechanical model does not provide the desired model closely resembling a realistic skin model. The reason being, for a skin application of interest the chemical and mechanical properties of interest may vary, which need to be considered while testing a formulation. Moreover, for some skin applications only a chemical model or only a mechanical model may be an appropriate choice. Thus, the method and system disclosed herein provides a flexibility to select a custom chemical and mechanical model and enables selection of both or either of the model as per the requirement defined for skin application of interest.

The embodiments herein provide a method and system for insilico design and testing of formulations using physiochemical skin model providing rapid, accurate and reliable testing.

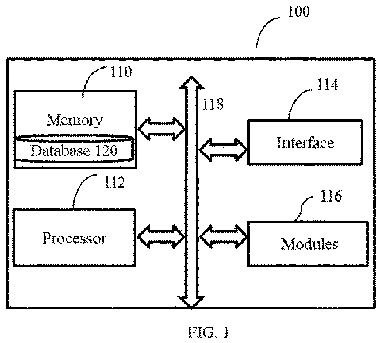

Referring now to the drawings, and more particularly to FIGS. 1 through 24, where similar reference characters denote corresponding features consistently throughout the figures, there are shown preferred embodiments and these embodiments are described in the context of the following exemplary system and/or method.

FIG. 1 is a block diagram of a system 100 for insilico design and testing of formulations using a physiochemical model of skin layer/s, interchangeably referred as physiochemical skin model, in accordance with some embodiments of the present disclosure.. As shown in FIG. 1, the system 100 includes one or more processor(s) 112 and a memory 110 communicatively coupled to each other. The system 100 also includes interface(s) 114 and modules 116. The modules 116 include a formulation module, a chemical model of the skin (not shown) and a mechanical model of the skin (not shown). The processor 112, the memory 110, the communication interface 114 and the sensors 116 may be coupled by a system bus such as a system bus 118 or a similar mechanism. Although FIG. 1 shows example components of the system, in other implementations, the system may contain fewer components, additional components, different components, or differently arranged components than depicted in FIG. 1.

The processor(s) 112, can be one or more hardware processors 112, and may include circuitry implementing, among others, audio and logic functions associated with the communication. The processor(s) 112 may include, among other things, a clock, an arithmetic logic unit (ALU) and logic gates configured to support operation of the processor(s) 112. The processor(s) 112 can be a single processing unit or a number of units, all of which include multiple computing units. The processor(s) 112 may be implemented as one or more microprocessors, microcomputers, microcontrollers, digital signal processors, central processing units, state machines, logic circuitries, and/or any devices that manipulate signals based on operational instructions. Among other capabilities, the processor(s) 112 is configured to fetch and execute computer-readable instructions and data stored in the memory 110.

The functions of the various elements shown in the figure, including any functional blocks labeled as "processor(s)", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional, and/or custom, may also be included.

The interface(s) 114 may include a variety of software and hardware interfaces, for example, interfaces for peripheral device(s), such as a keyboard, a mouse, an external memory, and a printer. The interface(s) 114 can facilitate multiple communications within a wide variety of networks and protocol types, including wired networks, for example, local area network (LAN), cable, etc., and wireless networks, such as Wireless LAN (WLAN), cellular, or satellite. For the purpose, the interface(s) 114 may include one or more ports for connecting the system to other devices.

The memory 110 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. The memory 110, may store any number of pieces of information, and data, used by the system to implement the functions of the system. The memory 110 may be configured to store information, data, applications, instructions or the like for enabling the system to carry out various functions in accordance with various example embodiments. Additionally, or alternatively, the memory 110 may be configured to store instructions which when executed by the processor(s) 112 causes the system to behave in a manner as described in various embodiments. The memory 110 may store any number of pieces of information and data used by the system 100 to implement various functions in accordance with various example embodiments. Additionally, or alternatively, the memory 110 may be configured to store instructions which when executed by the processor 112 causes the system 100 to behave in a manner as described in various embodiments. The communication interface(s) 114 can facilitate multiple communications within a wide variety of networks and protocol types, including wired networks, for example, local area network (LAN), cable, etc., and wireless networks, such as Wireless LAN (WLAN), cellular, or satellite.

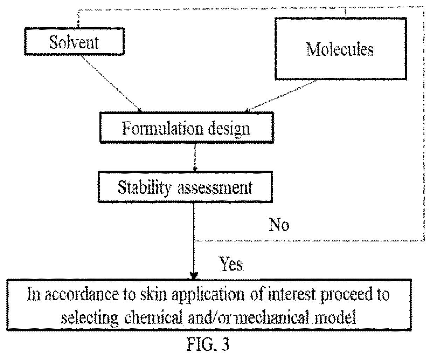

Further, the memory comprises a database 120. In an embodiment, the database 120 can be externally coupled to the system 100. The database120 comprises data corresponding to a plurality of chemical models, a plurality of mechanical models, a plurality of solvents, a plurality of active ingredients, a plurality of excipients and the like. The memory also comprises a formulations design module, the chemical model and the mechanical model, explained further in conjunction with FIGS. 3 through 5. The system 100 of the present subject matter studies the mechanical and chemical behavior of skin and its various microscopic components (collagen, elastin, lipid bilayer, etc.), on the application of a new designed formulation, at multiple length scales and integrates these studies to predict the enhanced behavior of skin. This aids in the complete design of a cosmetic/drug formulation for various cases such as skin ageing, collagen-based skin diseases and aesthetic skin appearances.

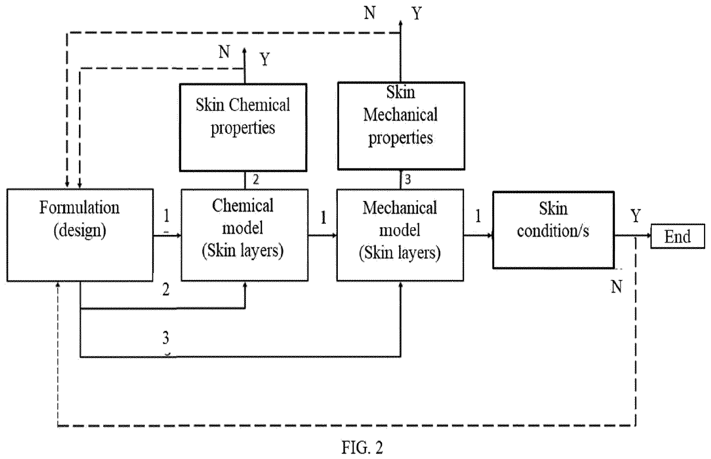

FIG. 2 is a functional block diagram of the system 100 of FIG. 1 illustrating insilico design and testing of formulations using physiochemical model of skin layer/s us, in accordance with some embodiments of the present disclosure. In one implementation, the present disclosure consists of the following components: (1) the formulations design module, (2) the chemical model interchangeably referred as the chemical model and (3) the mechanical model,.

(1) Formulations design module: In a cosmetic formulation, there are various constituents such as the active ingredients, carriers and different solvents (emulsifiers, stabilizers, moisturizers) that may serve different purposes. Typically, a solvent is used to dissolve or mix the active ingredients. An active ingredient is one, which shows the after effect once given in enough quantity (concentration), while carriers are molecules which by themselves do not provide any effect like active molecule but they interact with skin component and reduce the skin barrier so that active molecules can penetrate the skin. In said implementation, the formulations design module gives several possible combinations of formulations made up of various kind of molecules (organic, inorganic, biomolecules), particles (nano/micro particles), solvents etc. The module screens all possible combination of constituents (of formulation) and gives the stable formulation.

(2) Chemical model of the skin: As described above, skin has a chemical barrier, i.e., stratum corneum (SC) that helps in preventing moisture loss and external agents passing through the skin. For the formulation to function, it must pass through this barrier. The chemical model module aids in the permeation study of the cosmetic formulation through this barrier at molecular and macro levels. In an example embodiment, the molecular level properties of formulations were studied using molecular simulations. Molecular transport parameters are translated to macroscopic scale to study their release profiles through SC and other deeper layers as well. If the formulation fails to permeate, then redesigning of formulations is to be carried out using the preceding (formulations) module.

(3) Mechanical model of the skin: As known widely, dermis, the mechanical support of skin constitutes proteins such as collagen and elastin that provides support and suppleness to skin. From the existing studies, it is known that collagen is the most important component that influences skin appearance with respect to aging. Various formulations target in affecting collagen structure for providing anti-aging effects. The effect of formulation from the preceding module on the structural changes in the collagen molecule is studied using molecular simulations. The macroscopic properties are modelled from the molecular properties considering the hierarchical arrangement of collagen in the dermis layer.

Further, macroscopic techniques such as finite element method and polymer inspired constitutive modelling, etc., are used for estimating the bulk properties of dermis. Enhancement of the overall skin mechanical properties can occur even if the formulation does not bring structural changes in collagen. A thin layer of cosmetic on the skin can result in a change in the overall skin mechanical properties (skin + cosmetic layer) and this anti-ageing effect is also captured in this framework. If the expected mechanical properties (such as elasticity, relaxation time) are not enhanced, then optimization is to be carried out using the preceding module. At present, top-down approach has been employed owing to the inexpensive simulation costs to simulate the skin mechanical behavior. Currently, experimental data of whole skin are used in macroscopic constitutive modelling technique to estimate the behavior of skin on addition of a layer of cosmetic polymer to skin.

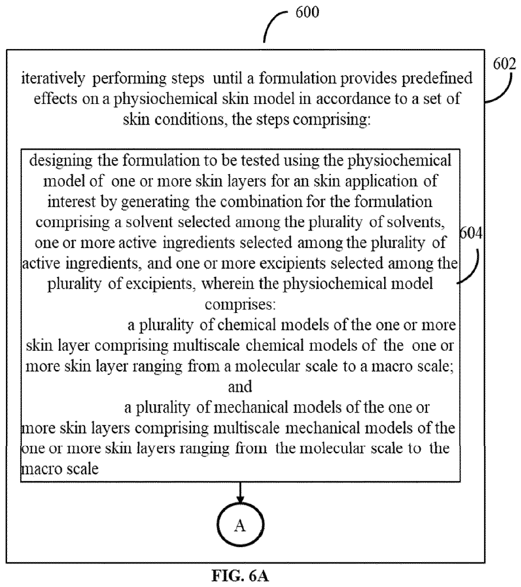

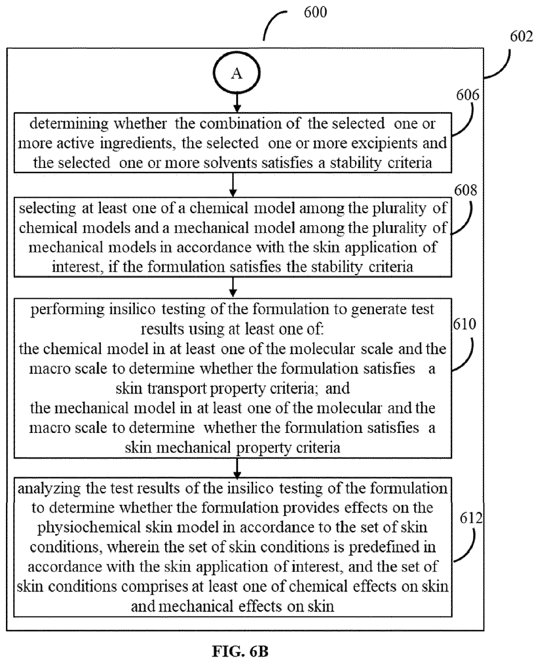

The above described method is further described in detail with help of functional block diagrams of FIGS. 2 through 5 and method steps of FIGS. 6A and 6B. Referring to FIGS. 2-5, the solid lines represent the workflow process in accordance in an embodiment of the present disclosure and the dashed lines represent the optimization loop where the process is repeated until the process follows the solid line pathway. FIG. 2 illustrates the overall schematic representation and FIGS. 3-5 describe the different routes of workflow that could be carried out according to various skin applications of interest for which a designed formulation has to be tested.

As shown in the FIG. 2, the workflow primarily evaluates the stability of the formulation using molecular principles. The stable formulation may then be evaluated for its permeability through the skin lipid bilayer. Thereafter release profile may be calculated from the chemical model at a macro scale, interchangeably referred herein as macroscopic skin chemical model, using the physical properties calculated from the chemical model at the micro scale, interchangeably referred as microscopic skin chemical model.

In one implementation, the formulation of known concentration may be tested by the mechanical model to observe the structural changes in the collagen molecule. The structural changes are then translated along with the hierarchical arrangement of collagen molecule to the mechanical model at the macro scale, interchangeably referred as macroscopic skin mechanical model. The formulation efficacy in terms of mechanical properties of skin were estimated. The modelling pathways 1,2 and 3 depicted in FIG 2 are mutually exclusive based on the interest of the problem (i.e.., Sequential pathway 1 is not mandatory)

FIG. 3 represents various blocks of the formulations design module of the system of FIG. 1 in detail and the steps carried out. In an implementation, the solvent/s (organic/in-organic/aqueous etc.) is any medium in which the active ingredient and carriers were dispersed. Herein the active ingredients may be biomolecules, chemical molecules (organic, inorganic, nanoparticles, biomolecules, etc.) that bring about the change in the structural property of skin.

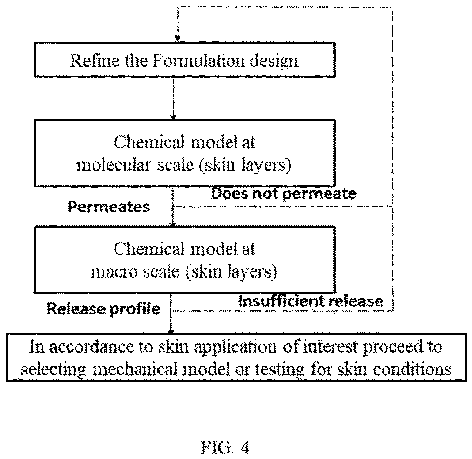

FIG. 4 represents a functional block diagram of the chemical model at micro scale and macro scale, where the formulation is assessed for its permeability through skin. For example, hydrophilic molecules and large molecules do not pass through the skin barrier and hence it is necessary to study the formulation's permeability through this barrier before evaluating the efficacy. If the molecule is permeable, the properties are translated to macroscopic model to study the release profiles.

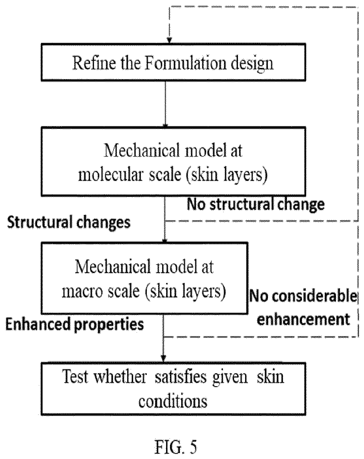

FIG. 5 represents a functional block diagram of the mechanical model of skin layer at micro scale and macro scale, where the formulation plays an active role. The formulation effectively alters the constituents of this layer. As before, the changes are translated to the macroscopic scale and efficacy of the molecule is determined by comparing the result with natural skin (without specification application of cosmetic)

FIGS. 6A and 6B depict a flow diagram illustrating a method 600 for insilico design and testing of skin formulations using the physiochemical model generated by the system 100 of FIG. 1, in accordance with some embodiments of the present disclosure. In an embodiment, the system 100 comprises one or more data storage devices or the memory 110 operatively coupled to the processor(s) 112 and is configured to store instructions for execution of steps of the method 600 by the processor (s) 112. The steps of the method 600 of the present disclosure will now be explained with reference to the components or blocks of the system 100 as depicted in FIG. 1 through FIG. 5 and the steps of flow diagram as depicted in FIGS. 6A and 6B. Although process steps, method steps, techniques or the like may be described in a sequential order, such processes, methods and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 602 of the method 600, the processor(s) 112 is configured to iteratively perform steps until a formulation provides predefined or desired effects on a physiochemical skin model in accordance to a set of skin conditions. The formulation is a combination among a plurality of combinations, which can be generated from a plurality of active ingredients, a plurality of excipients and a plurality of solvents. The desired or the predefined effects herein refer to amount of active ingredient, which is permeating across the skin layer. Any active molecule should reach to a minimum quantity to show its effect on skin properties. Hence, the method disclosed enables changing the formulation component (cosolvent, carriers, etc.) until active permeates in the skin to a desired quantity.

The plurality of solvents include aqueous solvents, non-aqueous solvents and organic solvents and the like. The plurality of active ingredients comprise therapeutic molecules, drug molecules, potential molecules, nanomaterials and biomaterials and the like. The plurality of excipients comprise nanomaterials, biomolecules, permeation enhancers, organic molecules and inorganic molecules and the like.

The iterative steps are preformed to obtain an appropriate formulation for appropriate results for a skin application and are described below with help of steps 604 through 612. The skin application of interest can be from domain of healthcare solutions or personal care solutions such as antiaging effects, on skin and the like.

At step 604 of method 600 the one or more hardware processors 112 are configured to design the formulation to be tested. The testing of the formulation is performed using the physiochemical model of one or more skin layers for a skin application of interest. The design formulation module facilitates design to generate the combination (chemical combination) for the formulation, wherein the combination comprises a solvent selected among the plurality of solvents, one or more active ingredients selected among the plurality of active ingredients, and one or more excipients selected among the plurality of excipients. Thus, system 100 provides options to redesign/ refine a selected formulation till it provides a desired result on the skin physiochemical model. The physiochemical model as mentioned earlier is an integrated model providing multiple models at various scales. Thus, the method and system provides:

1. a plurality of chemical models of the one or more skin layers. The chemical model comprises multiscale chemical models of the one or more skin layer ranging from a molecular scale to a macro scale; and
2. a plurality of mechanical models of the one or more skin layers comprising multiscale mechanical models of the one or more skin layers ranging from the molecular scale to the macro scale.

Once the formulation is generated, at step 606 of method 600, the one or more hardware processors 112 are configured to determine whether the combination created by the selected one or more active ingredients, the selected one or more excipients and the selected one or more solvents satisfies a stability criteria. The stability criteria can be preset in accordance with known stability requirements for a formulation for the skin application of interest. The stability criteria indicates that when a selected solvent, a selected carrier and a selected active molecule for redesigning the formulation are mixed together they should not agglomerate or phase separate. In case they agglomerate or phase separate, the formulation design is not stable and fails to pass the stability criteria test.

Once the formulation satisfies the stability criteria, an appropriate chemical or a mechanical model or a combination thereof, which is customized for the skin application of interest is selected. Thus, at step 608 at least one of a chemical model among the plurality of chemical models and a mechanical model among the plurality of mechanical models is selected. The customized models are selected from a repository of chemical and mechanical models available to the system 100 or that may be stored in the database 120. The chemical models and the mechanical models molecular to macro level are available. The selection of model from the repository is dependent on the content in the designed formulation. This model mapping may be predefined by a subject matter expert.

Further, at step 610 of method 600, the one or more hardware processors 112 are configured to perform insilico testing of the designed formulations performed using the selected chemical or the mechanical model or the combination of the chemical model and the mechanical model:

The chemical model is used at the molecular scale and/or the macro scale to determine whether the formulation satisfies a skin transport property criteria. The skin transport property refers to parameters of the skin such as permeability, or release profiles or the like. The testing of formulation using the chemical model is performed at molecular scale to check whether a permeation property of the formulation satisfies a permeability criteria. Further, if the permeability criteria is satisfied by the formulation under test, the chemical model at macro scale is used during testing to determine whether a release profile of the formulation satisfies a release criteria. The permeability criteria is preset in accordance with preset permeation threshold , and the release criteria is preset in accordance to a preset therapeutic window.

Once the testing of the formulation is carried out on the chemical model, the mechanical model and the combination, at step 612 of method 600, the one or more hardware processors are configured to analyze the test results of the insilico testing of the formulation to determine whether the formulation provides effects on the physiochemical skin model in accordance to the set of skin conditions. The set of skin conditions is predefined in accordance with the skin application of interest, and the set of skin conditions comprises at least one of chemical effects on skin and mechanical effects on skin.

**Experimental results:** The system 100 disclosed herein is tested against the available experimental data. The chemical model is tested at both molecular and macroscopic levels and the mechanical model is tested in terms of macroscopic modelling perspective. In the experiment, instead of molecular simulations, the parameters are obtained from experimental data to model skin mechanical behavior. In this embodiment, the elasticity and relaxation times of skin (various ages and location) were calculated and validated with various experimental studies. Following experimental data describes the results of 1D-Viscoelastic model for behavior of skin with respect to age and location. Two case studies were taken where the skin is subjected to creep and distensibility (herein the experimental data is obtained from Krueger et al and Yu et al).

**First case study:** In the first case study, Krueger et al had conducted a cutometric *in vivo* test to observe mechanical property changes of 120 female subjects with respect to age and location. In this case study, 120 female subjects were categorized evenly into four classes according to the age groups as follows, Group I: 18-29 years, Group II: 30-39 years, Group III: 40-49 years, Group IV: 50-65 years. A constant stress of 450 mbar was applied at five different locations (Thickness of skin is taken to be 1 mm). The displacement data as obtained from reference was converted to strain data by considering the above mentioned skin thickness. The strain behavior with respect to time was studied for 2 s suction (loading) and 2 s relaxation period.

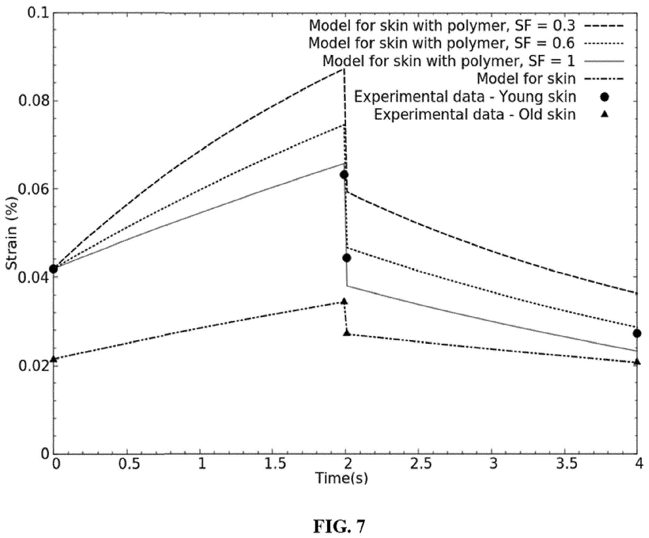

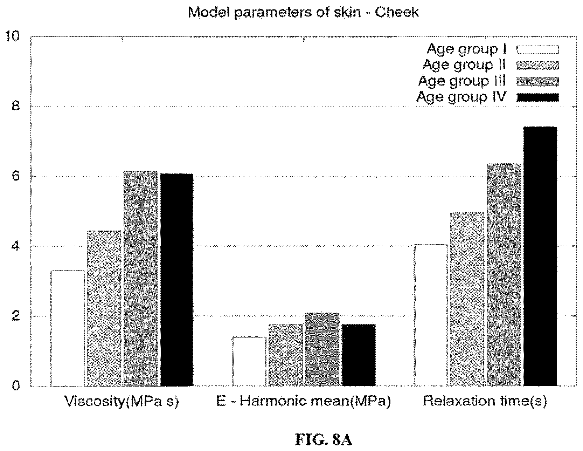

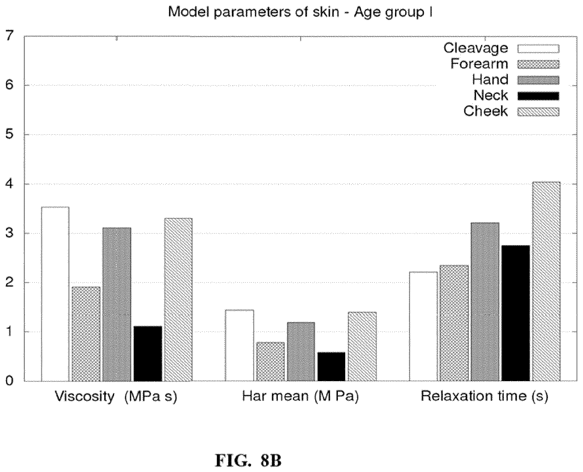

FIG. 7 illustrates a Generalized Voigt model explaining the creep behavior of skin. The mechanical properties obtained from the developed Generalized Voigt model using the experimental data obtained from literature, correlate well with age, and may be used to understand the microscopic origins of ageing. The model parameters with respect to location and the values are as shown in the FIG. 8A and 8B respectively. From the FIG. 8A, it is evident that the increase in viscosity with age is more evident than the change in elasticity.

**Second case study:** In order to validate the modeling framework further, second case study by Yu et al, was chosen wherein vivo mechanical experiments were carried out on skin in the presence of a second layer of cross-linked polymer layer (XPL). In this study, target values of second skin polymer composition were obtained from experimental study on excised human skin by Annaidh et al. The mechanical response of the skin was measured by Yu et al to assess the effect of the crosslinking polymer that was synthesized. In the second case study, the volar arm skin of women aged 40 and above is distended to 12 % strain and is released.

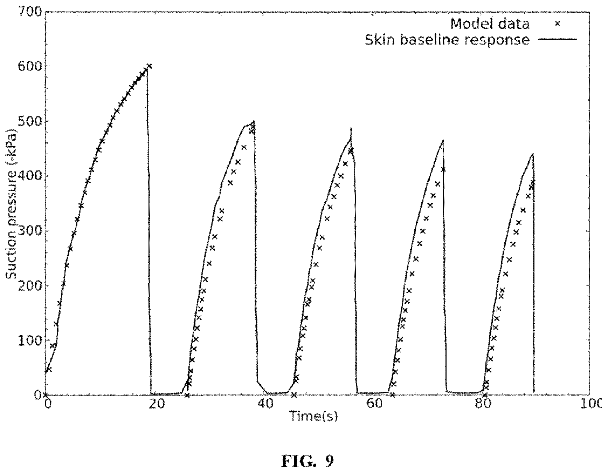

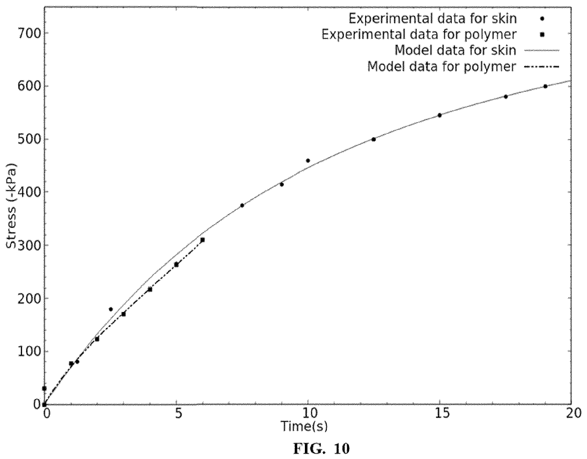

The negative pressure in the suction device was monitored with time until 12 % strain is achieved, and this was repeated for 5 consecutive cycles as depicted by the skin baseline data in FIGS. 9. As shown in FIG. 9, the model shows good agreement with experimental data for skin with and without polymer. The hysteretic behavior that is observed in the study over a number of cycles is explained in the physiochemical model by collagen damage that occurs when the skin is subjected to mechanical forces. From this study, the viscosity from cutometric data can be estimated, which is an important property, observed to change with age at a rate higher than that of the change in elasticity and is crucial in designing anti-ageing formulations. The stress behavior for a single cycle (first) is shown in FIG. 10 where the model represents the behavior of the skin with and without polymer using Generalized Maxwell Model

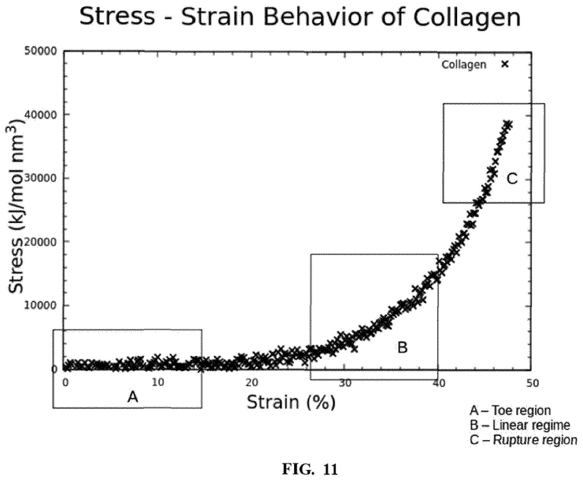

FIG. 11 represents the Stress-Strain behavior of a skin protein (Collagen) at the molecular level using molecular dynamics simulations where the structure (Type III collagen model) is extended until it ruptures. The different stages of material deformation such as the toe (A), linear (B) and rupture (C) regimes are depicted in the FIG. 11

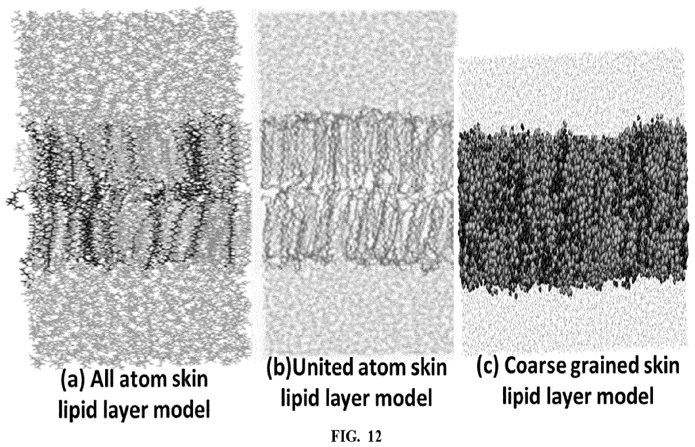

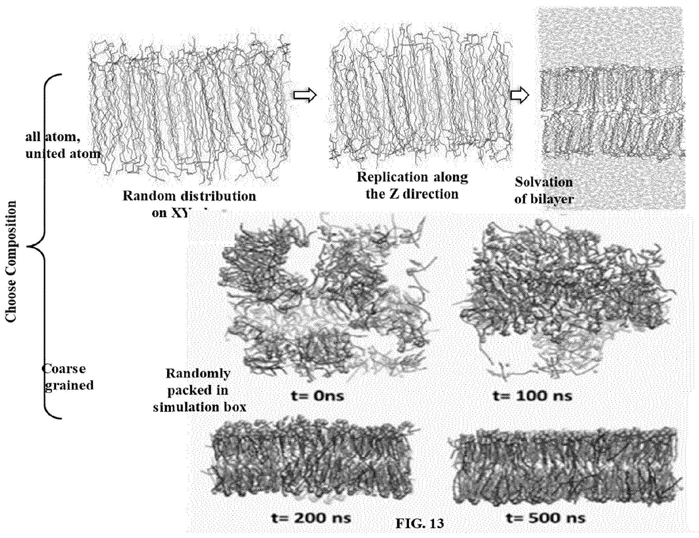

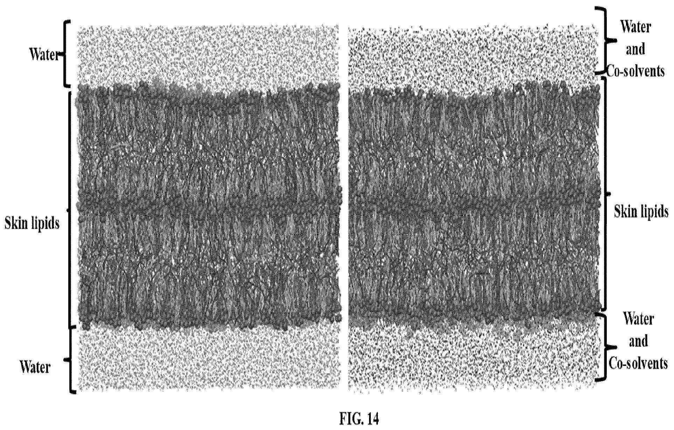

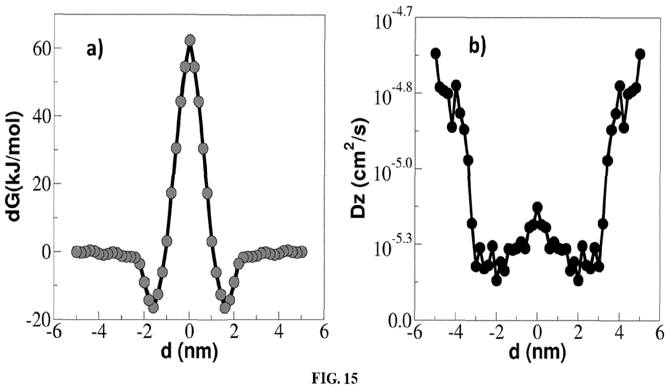

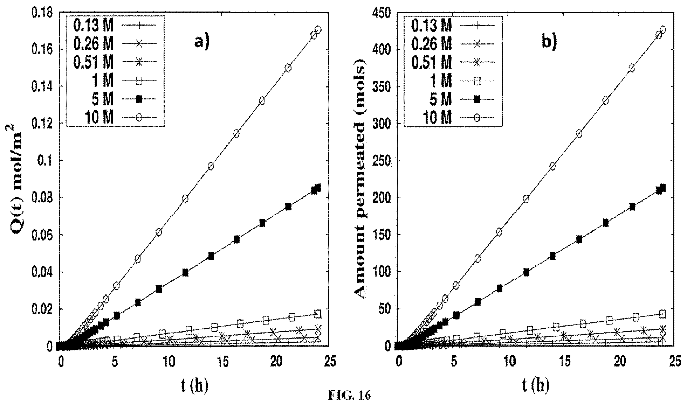

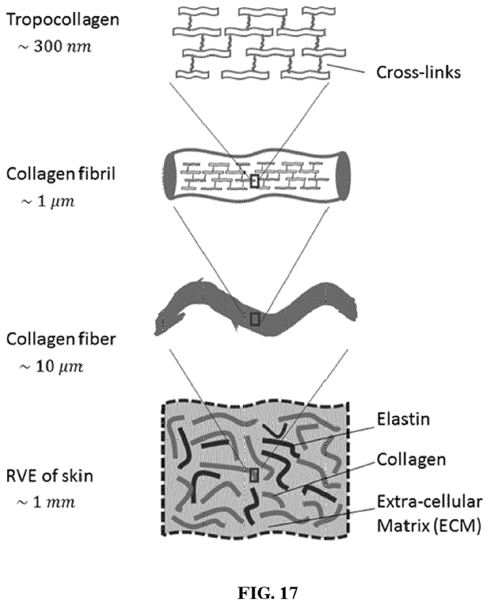

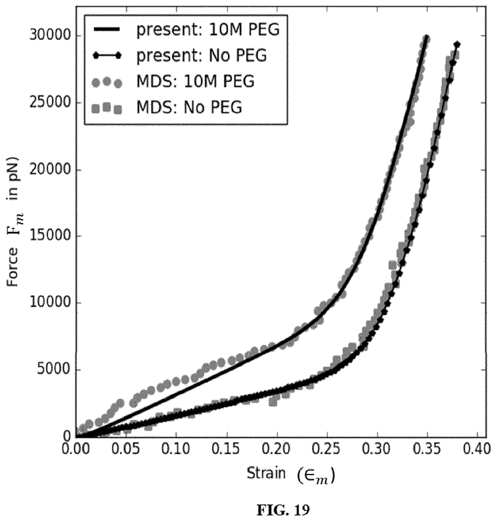

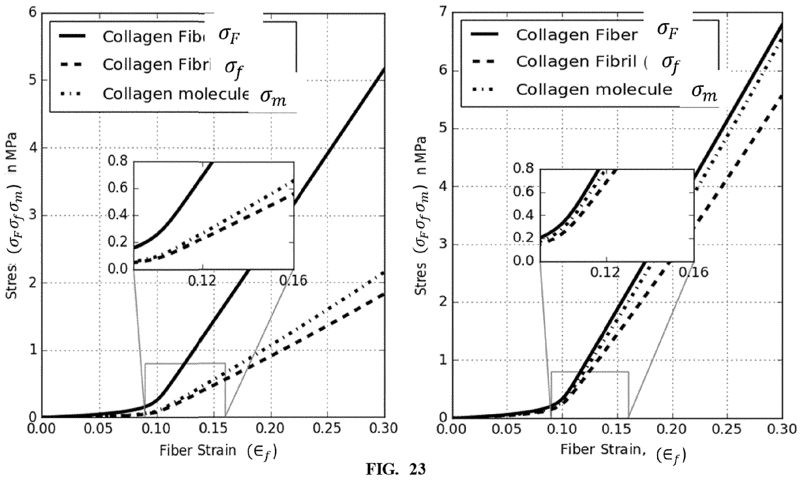

FIG. 12 depicts example skin lipid layer chemical model in the repository built using equimolar ratio of ceramides, cholesterol and free fatty at various levels comprising all atom, united atom and coarse grained, which provide molecular scale to macro scale chemical models. For example, while testing a formulation comprising ethanol, which is used as permeation enhancer, an all atom model is required. However, if the formulation comprises nano particles, a coarse grained model of the skin layers is necessary to be used during simulation. FIG.13 depicts methodology to develop skin lipid layer models at all atom, united atom and coarse grained levels. FIG. 14 depicts skin multi lipid layer model (chemical models), wherein in FIG. 14, a) represents stratum corneum lipid environment and b) mimics the deeper layer environment of viable epidermis by adding the co-solvent. FIG. 15 depicts simulation results provided by a molecular scale chemical model of the system 100 for an example formulation such as polyethylene glycol (PEG). FIG 15(a) depicts free energy of PEG across the skin SC lipid bilayer, while FIG 15 (b) depicts diffusivity of PEG at various location inside the skin SC lipid bilayer. The free energy and diffusivity are calculated by constrained molecular dynamics simulations. FIG. 16 depicts macro scale chemical model, wherein (a) depicts cumulative release profile from stratum corneum for different concentrations of PEG in a vehicle (b) depicts mass of PEG permeated into dermis for different concentrations of PEG in vehicle. The properties are calculated using multiscale model of skin SC layer. The mechanical model is also used at the molecular scale and/or the macro scale to determine whether the formulation satisfies a skin mechanical property criteria. For example, the mechanical model at the macro scale is used if the formulation does not permeate inside the skin and changes the overall mechanical properties of the skin. In case, the formulation permeates through the skin and changes the molecular properties, then a molecular scale model is used. The mechanical property refers to parameters of the skin such as structural changes, skin enhancements. The molecular scale mechanical model is used to determine whether structural changes in the mechanical model due to the formulation satisfy a structural change criteria. The structural change criteria may refer to solvation around collagen molecule that leads to change in mechanical properties of the skin. Further, the mechanical model at the macro scale is used to determine whether enhancements in the mechanical model due to the formulation satisfy an enhancement criteria. The enhancement criteria, for example, would be anti-ageing effects, which are correlated with mechanical properties like elasticity and viscoelastic relaxation time constant. The structural change criteria and the enhancement criteria are preset by experts in accordance with the skin application of interest. FIG. 17 illustrates hierarchal or multiscale arrangement of skin dermis component (collagen), which is responsible for skin's viscoelastic or mechanical properties. This is a typical skin's epidermis structure at various scales (molecular level to macroscopic level). The multiscale elastic model of collagens is built in accordance with works carried out in literature to extend the mechanical properties obtained from molecular scale to macroscale. In accordance with the study in the literature the low elongation regime mechanical response is governed by Entropic elasticity. The mechanical behavior of molecules can be described by Worm Like Chain (WLC) models in entropic regime. Beyond contour length WLC alone is not sufficient to calculate the end to end extension of molecules When molecular extension approaches molecular contour length, the applied force contributes to activate the stretch of molecular covalent bonds, inducing the onset of energetic mechanisms. The mechanical properties corresponding to various length scales (molecule, fibril and fiber) are shown in FIGS. 19 and 23.

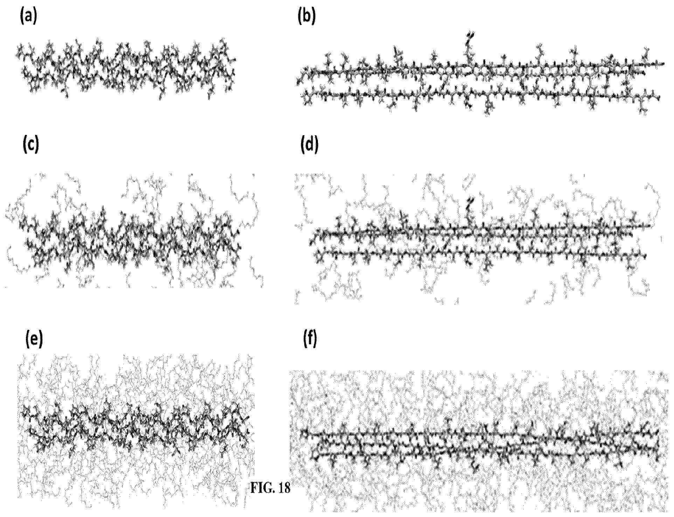

FIG. 18 illustrates simulation results of system of FIG. 1 indicating structural changes to collagen molecules when stress is applied, in accordance to some embodiments of the present disclosure. The FIG. 18 depicts simulation results provided by a selected mechanical model of the system 100, wherein FIG. 18 (a and ,b) indicates stretching of a single collagen molecule in absence. FIG 18 (c and d) depicts presence of the dehydrating agent PEG: 1M PEG and FIG. 18 (e and f) is with :10M PE). Further, FIG. 18 (a, c and e) shows the initial structure before stretching and FIG. 18 (b, d and f) shows final structure having strain ~35 % due to stretching at a loading rate of 0.01 nm/ps. The helical structure gradually unfolds with increasing strain and vanishes at large deformation. In the presence of PEG, there is an increased resistance towards unfolding of helical structure as shown in FIG. 18 (g and f), where the three chains of the molecule are more entangled to each other as compared with FIG. 18 (b and d).

FIG. 19 depicts force-strain diagram of collagen molecule, with and without PEG, compared with molecular dynamics simulation(MDS) with reference to FIG. 18. Table 1 below represents the parameters used for simulating nanoscale collagen mechanics in the presence (10 M concentration) and absence of PEG.

TABLE: 1

| Parameters | No PEG | 10M PEG |
|---|---|---|
| $T$ | 310 K | 310 K |
| $l_p$ | 16 $nm$ | 16 $nm$ |
| $l_c$ | 9.25 $nm$ | 8.98 $nm$ |
| $l_{m,o}$ | 9.145 $nm$ | 9.1 $nm$ |
| $A_m$ | 3 $nm^2$ | 2.25 $nm^2$ |

(continued)

| Parameters | No PEG | 10M PEG |
|---|---|---|
| $\hat{E}_0$ | 6 GPa | 16 GPa |
| $\hat{E}$ | 120 GPa | 130 GPa |
| $\eta$ | 45 | 45 |
| $\varepsilon_h^0$ | 0.3 | 0.27 |
| $\mu$ | 0.85 | 0.85 |

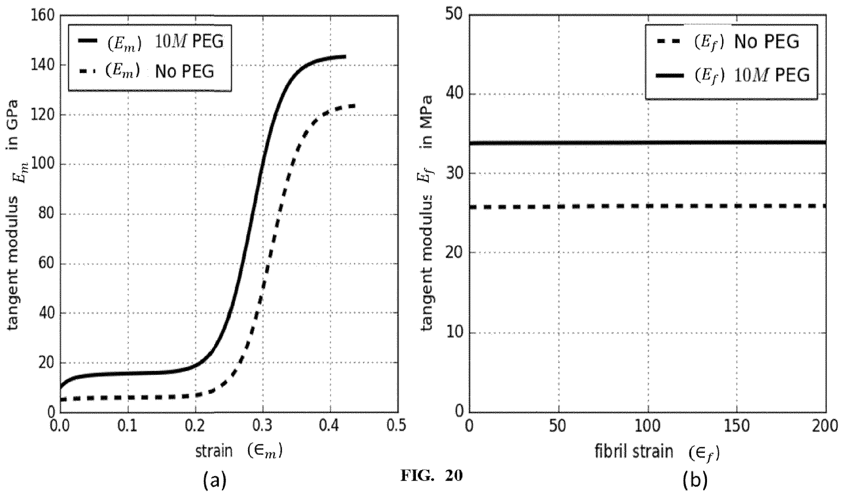

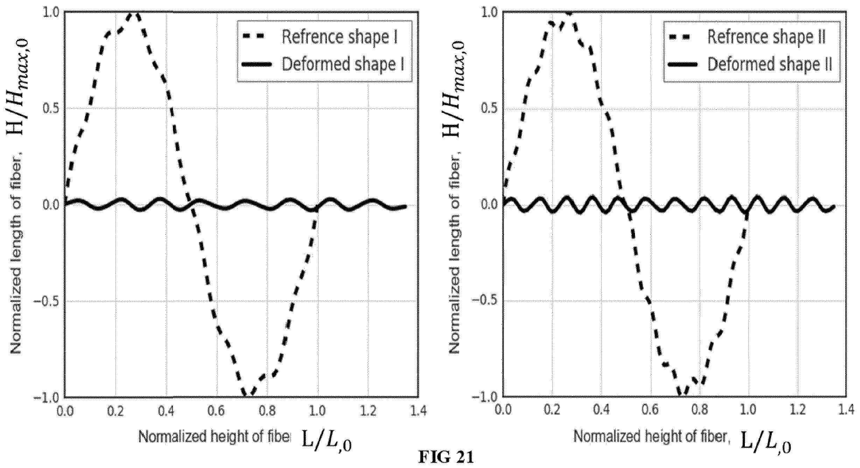

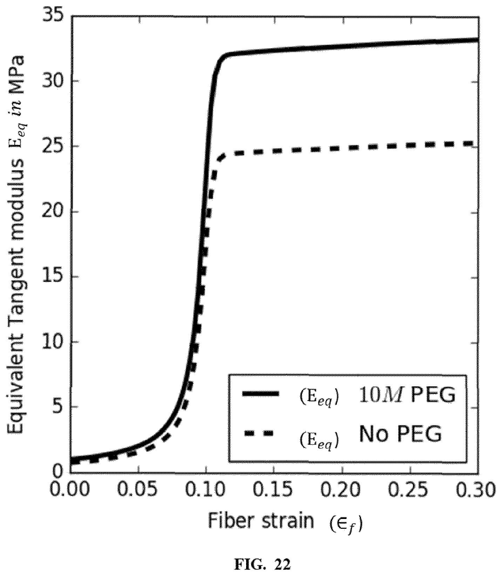

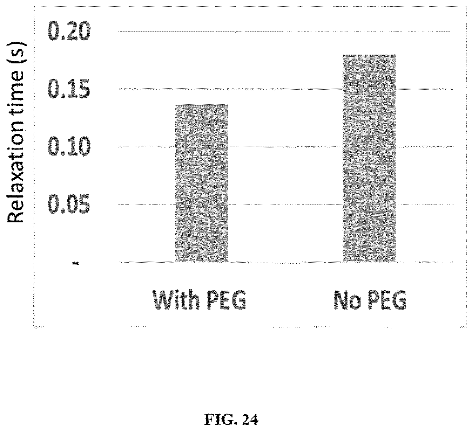

FIG. 20 depicts variation of tangent modulus of (a) collagen molecule vs molecular strain, (b) collagen fibril vs fibril strain, for PEG and without PEG. FIG. 21 depicts deformation of collagen fiber of two different initial shape, dotted lines shows the intact while solid lines for deformed fiber. FIG. 22 depicts variation of Equivalent tangent modulus of collagen fiber for PEG and without PEG. FIG. 23 depicts stress-strain behavior of collagen fiber, fibril and molecule for (a.) No PEG (b) 10 M PEG. FIG. 24 depicts relaxation time (in seconds) of skin calculated based on the fiber tangent modulus with PEG and without PEG.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method for insilico design and testing of formulations, the method comprising:
iteratively performing (602) steps, by one or more hardware processors until a formulation provides predefined effects on a physiochemical skin model in accordance to a set of skin conditions, wherein the formulation is a combination among a plurality of combinations generated from a plurality of active ingredients, a plurality of excipients, and a plurality of solvents, the steps comprising:

designing (604) the formulation, by the one or more hardware processors, to be tested using the physiochemical model of one or more skin layers for a skin application of interest, wherein the step of designing comprises generating the combination for the formulation comprising a solvent selected among the plurality of solvents, one or more active ingredients selected among the plurality of active ingredients, and one or more excipients selected among the plurality of excipients , wherein the physiochemical model comprises:

a plurality of chemical models of the one or more skin layers comprising multiscale chemical models of the one or more skin layers ranging from a molecular scale to a macro scale; and
a plurality of mechanical models of the one or more skin layers comprising multiscale mechanical models of the one or more skin layers ranging from the molecular scale to the macro scale;

determining (606), by the one or more hardware processors, whether the combination of the selected one or more active ingredients, the selected one or more excipients and the selected one or more solvents satisfies a stability criteria, wherein the combination is modified if the stability criteria is not satisfied;
selecting (608) at least one of a chemical model among the plurality of chemical models and a mechanical model among the plurality of mechanical models, by the one or more hardware processors, in accordance with the skin application of interest, if the formulation satisfies the stability criteria;
performing (610) an insilico testing of the formulation, by the one or more hardware processors, to generate test results using at least one of:

the chemical model in at least one of the molecular scale and the macro scale to determine whether the formulation satisfies a skin transport property criteria; and
the mechanical model in at least one of the molecular scale and the macro scale to determine whether the formulation satisfies a skin mechanical property criteria; and

analyzing (612), by the one or more hardware processors, the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

2. The method as claimed in claim 1, wherein performing the insilico testing of the formulation to generate the test results using the chemical model at the molecular scale and the macro scale to determine whether the formulation satisfies the skin transport property criteria comprises:

determining, using the chemical model at molecular scale, whether a permeation property of the formulation satisfies a permeability criteria; and
determining, using the chemical model at the macro scale, whether a release profile of the formulation satisfies a release criteria, wherein the chemical model at the macro scale is applied if the formulation satisfies the permeability criteria.

3. The method as claimed in claim 1, wherein performing the insilico testing of the formulation to generate the test results using the mechanical model at the molecular scale and the macro scale to determine whether the formulation satisfies the skin mechanical property criteria comprises:

determining, using the mechanical model at the molecular scale, whether structural changes in the mechanical model due to the formulation satisfy a structural change criteria; and
determining, using the mechanical model at the macro scale, whether enhancements in the mechanical model due to the formulation satisfy an enhancement criteria.

4. The method as claimed in claim 1, wherein the chemical model is customized for the skin application of interest by selecting the chemical model from a repository of chemical models.

5. The method as claimed in claim 1, wherein the mechanical model is customized for the skin application of interest by selecting the mechanical model from a repository of mechanical models.

6. The method as claimed in claim 1, wherein

the plurality of solvents comprise at least one of aqueous solvents, non-aqueous solvents and organic solvents,
wherein the plurality of active ingredients comprise at least one of therapeutic molecules, drug molecules, potential molecules, nanomaterials and biomaterials; and
wherein the plurality of excipients comprise at least one of nanomaterials, biomolecules, permeation enhancers, organic molecules and inorganic molecules.

7. A system (100) for insilico design and testing of formulations., the system (100) comprising:

a memory (110) storing instructions;
one or more Input/Output (I/O) interfaces (114); and
a processor(s) (112) coupled to the memory (110) via the one or more I/O interfaces (114), wherein the processor(s) (112) is configured by the instructions to:
iteratively perform steps until a formulation provides predefined effects on a physiochemical skin model in accordance to a set of skin conditions, wherein the formulation is a combination among a plurality of combinations generated from a plurality of active ingredients, and a plurality of excipients, a plurality of solvents , the steps comprising:
designing the formulation to be tested using the physiochemical model of one or more skin layers for a skin application of interest, wherein the step of designing comprises generating the combination for the formulation comprising a solvent selected among the plurality of solvents, one or more active ingredients selected among the plurality of active ingredients, and one or more excipients selected among the plurality of excipients, wherein the physiochemical model comprises:

a plurality of chemical models of the one or more skin layer comprising multiscale chemical models of the one or more skin layer ranging from a molecular scale to a macro scale; and
a plurality of mechanical models of the one or more skin layers comprising multiscale mechanical models of the one or more skin layers ranging from the molecular scale to the macro scale;

determining whether the combination of the selected one or more active ingredients, the selected one or more excipients and the selected one or more solvents satisfies a stability criteria, wherein the combination is modified if the stability criteria is not satisfied;

selecting at least one of a chemical model among the plurality of chemical models and a mechanical model among the plurality of mechanical models in accordance with the skin application of interest, if the formulation satisfies the stability criteria;

performing an insilico testing of the to generate test results using at least one of:

the chemical model in at least one of the molecular scale and the macro scale to determine whether the formulation satisfies a skin transport property criteria; and
the mechanical model in at least one of the molecular and the macro scale to determine whether the formulation satisfies a skin mechanical property criteria;

analyzing the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

8.  The system (100) as claimed in claim 7, wherein the one or more hardware processors (112) are configured to perform the insilico testing of the formulation, to generate the test results using the chemical model at the molecular scale and the macro scale to determine whether the formulation satisfies the skin transport property criteria, by:

determining, using the chemical model at molecular scale, whether a permeation property of the formulation satisfies a permeability criteria; and
determining, using the chemical model at the macro scale, whether a release profile of the formulation satisfies a release criteria, wherein chemical model at macro scale is applied if the formulation satisfies the permeability criteria.

9.  The system (100) as claimed in claim 7, wherein the one or more hardware processors (112) are configured to perform the insilico testing of the formulation, to generate the test results using the mechanical model at the molecular scale and the macro scale to determine whether the formulation satisfies the skin mechanical property criteria, comprises:

determining, using the mechanical model at the molecular scale, whether structural changes in the mechanical model due to the formulation satisfy a structural change criteria;
determining, using the mechanical model at the macro scale, whether enhancements in the mechanical model due to the formulation satisfy an enhancement criteria.

10. The system (100) as claimed in claim 7, wherein the chemical model is customized for the skin application of interest by selecting the chemical model from a repository of chemical models.

11. The system (100) as claimed in claim 7, wherein the mechanical model is customized for the skin application of interest by selecting the mechanical model from a repository of mechanical models.

12. The system (100) as claimed in claim 7, wherein

the plurality of solvents comprise at least one of aqueous solvents, non-aqueous solvents and organic solvents;
wherein the plurality of active ingredients comprise at least one of therapeutic molecules, drug molecules, potential molecules, nanomaterials and biomaterials; and
wherein the plurality of excipients comprise at least one of nanomaterials, biomolecules, permeation enhancers, organic molecules and inorganic molecules.

13. One or more non-transitory machine readable information storage mediums comprising one or more instructions, which when executed by one or more hardware processors causes a method, the method comprising
iteratively performing steps until a formulation provides predefined effects on a physiochemical skin model in accordance to a set of skin conditions, wherein the formulation is a combination among a plurality of combinations generated from a plurality of active ingredients, a plurality of excipients, and a plurality of solvents, the steps comprising:

designing to be tested using the physiochemical model of one or more skin layers for a skin application of interest, wherein the step of designing comprises generating the combination for the formulation comprising a solvent selected among the plurality of solvents, one or more active ingredients selected among the plurality of active ingredients, and one or more excipients selected among the plurality of excipients, wherein the physiochemical model comprises:

a plurality of chemical models of the one or more skin layers comprising multiscale chemical models of the one or more skin layers ranging from a molecular scale to a macro scale; and
a plurality of mechanical models of the one or more skin layers comprising multiscale mechanical models of the one or more skin layers ranging from the molecular scale to the macro scale;

determining whether the combination of the selected one or more active ingredients, the selected one or more excipients and the selected one or more solvents satisfies a stability criteria, wherein the combination is modified if the stability criteria is not satisfied;
selecting at least one of a chemical model among the plurality of chemical models and a mechanical model among the plurality of mechanical models in accordance with the skin application of interest, if the formulation satisfies the stability criteria;
performing an insilico testing of the formulation to generate test results using at least one of:

the chemical model in at least one of the molecular scale and the macro scale to determine whether the formulation satisfies a skin transport property criteria; and
the mechanical model in at least one of the molecular scale and the macro scale to determine whether the formulation satisfies a skin mechanical property criteria;

analyzing the test results of the insilico testing of the formulation to determine whether the formulation provides the predefined effects on the physiochemical skin model in accordance to the set of skin conditions, wherein the set of skin conditions is predefined in accordance with the skin application of interest, and the set of skin conditions comprises at least one of chemical effects on skin and mechanical effects on skin.

FIG. 1

FIG. 2

FIG. 3

Refine the Formulation design

Chemical model at
molecular scale (skin layers)

**Permeates** — **Does not permeate**

Chemical model at
macro scale (skin layers)

**Release profile** — **Insufficient release**

In accordance to skin application of interest proceed to
selecting mechanical model or testing for skin conditions

FIG. 4

Refine the Formulation design

Mechanical model at
molecular scale (skin layers)

**No structural change**

**Structural changes**

Mechanical model at
macro scale (skin layers)

**No considerable
enhancement**

**Enhanced properties**

Test whether satisfies given skin
conditions

FIG. 5

600

602

iteratively performing steps until a formulation provides predefined effects on a physiochemical skin model in accordance to a set of skin conditions, the steps comprising:

604

designing the formulation to be tested using the physiochemical model of one or more skin layers for an skin application of interest by generating the combination for the formulation comprising a solvent selected among the plurality of solvents, one or more active ingredients selected among the plurality of active ingredients, and one or more excipients selected among the plurality of excipients, wherein the physiochemical model comprises:

a plurality of chemical models of the one or more skin layer comprising multiscale chemical models of the one or more skin layer ranging from a molecular scale to a macro scale; and

a plurality of mechanical models of the one or more skin layers comprising multiscale mechanical models of the one or more skin layers ranging from the molecular scale to the macro scale

A

**FIG. 6A**

600

602

A

determining whether the combination of the selected one or more active ingredients, the selected one or more excipients and the selected one or more solvents satisfies a stability criteria

606

selecting at least one of a chemical model among the plurality of chemical models and a mechanical model among the plurality of mechanical models in accordance with the skin application of interest, if the formulation satisfies the stability criteria

608

performing insilico testing of the formulation to generate test results using at least one of:
the chemical model in at least one of the molecular scale and the macro scale to determine whether the formulation satisfies a skin transport property criteria; and
the mechanical model in at least one of the molecular and the macro scale to determine whether the formulation satisfies a skin mechanical property criteria

610

analyzing the test results of the insilico testing of the formulation to determine whether the formulation provides effects on the physiochemical skin model in accordance to the set of skin conditions, wherein the set of skin conditions is predefined in accordance with the skin application of interest, and the set of skin conditions comprises at least one of chemical effects on skin and mechanical effects on skin

612

**FIG. 6B**

**FIG. 7**

Model parameters of skin - Cheek

**FIG. 8A**

Model parameters of skin - Age group I

FIG. 8B

FIG. 9

**FIG. 10**

EP 3 683 800 A1

Stress - Strain Behavior of Collagen

FIG. 11

(a) All atom skin lipid layer model

(b) United atom skin lipid layer model

(c) Coarse grained skin lipid layer model

FIG. 12

FIG. 13

FIG. 14

FIG.15

FIG. 16

Tropocollagen
~ 300 nm

Cross-links

Collagen fibril
~ 1 μm

Collagen fiber
~ 10 μm

RVE of skin
~ 1 mm

Elastin

Collagen

Extra-cellular
Matrix (ECM)

FIG. 17

FIG. 18

FIG. 19

FIG. 20

**FIG 21**

**FIG. 22**

FIG. 23

EP 3 683 800 A1

**FIG. 24**

Europäisches
Patentamt

European
Patent Office

Office européen
des brevets

**EUROPEAN SEARCH REPORT**

Application Number

EP 20 15 2376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 370 178 A2 (TATA CONSULTANCY SERVICES LTD [IN]) 5 September 2018 (2018-09-05) * abstract * * paragraphs [0002], [0020], [0037]; claims 1,3,8,10,13 * | 1-13 | INV. G16H50/50 |
| A | COSTA RAQUEL ET AL: "Delivery systems for cosmetics - From manufacturing to the skin of natural antioxidants", POWDER TECHNOLOGY - ELECTROSTATIC PHENOMENA IN PARTICULATE PROCESSES, vol. 322, 2 August 2017 (2017-08-02), pages 402-416, XP085226284, ISSN: 0032-5910, DOI: 10.1016/J.POWTEC.2017.07.086 * abstract * * page 405, column 1, paragraph 3 * * page 411, column 1, paragraph 1 - paragraph 2 * | 1-13 | |
| A | FLYNN C ET AL: "Simulating the wrinkling and aging of skin with a multi-layer finite element model", JOURNAL OF BIOMECHANICS, PERGAMON PRESS, NEW YORK, NY, US, vol. 43, no. 3, 10 February 2010 (2010-02-10), pages 442-448, XP026867510, ISSN: 0021-9290 [retrieved on 2009-11-03] * abstract * | 1-13 | TECHNICAL FIELDS SEARCHED (IPC) G16H |

The present search report has been drawn up for all claims

| Place of search | Date of completion of the search | Examiner |
|---|---|---|
| Munich | 29 May 2020 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 15 2376

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2020

| Patent document cited in search report | | Publication date | Patent family member(s) | | Publication date |
|---|---|---|---|---|---|
| EP 3370178 | A2 | 05-09-2018 | AU | 2018201366 A1 | 20-09-2018 |
| | | | CN | 108536995 A | 14-09-2018 |
| | | | EP | 3370178 A2 | 05-09-2018 |
| | | | JP | 6591586 B2 | 16-10-2019 |
| | | | JP | 2018152058 A | 27-09-2018 |
| | | | KR | 20180101187 A | 12-09-2018 |
| | | | US | 2018253525 A1 | 06-09-2018 |

EPO FORM P0459

For more details about this annex : see Official Journal of the European Patent Office, No. 12/82

**EP 3 683 800 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201921002217